# EUROPEAN PATENT APPLICATION

(11) **EP 4 670 735 A1**
(43) Date of publication of application: **31.12.2025**
(21) Application number: 24183965.3
(22) Date of filing: 24.06.2024
(51) Int. Cl.: A61K 41/00, A61K 47/69, A61P 35/00

(54) **PRODRUG NANOPARTICLE FOR TUMOR-TARGETED ULTRASOUND-ACTIVIATED CANCER THERAPY**

(71) Applicant: Schleuning, Christian, 22851 Norderstedt (DE)
(72) Inventor: Karges, Johannes, 44791 Bochum (DE)
(74) Representative: Stork Bamberger Patentanwälte PartmbB

(57) **Abstract**

A prodrug nanoparticle for targeted accumulation and activation (treatment) in tumor tissue, that consists of a Pt(IV) complex with the following formula: 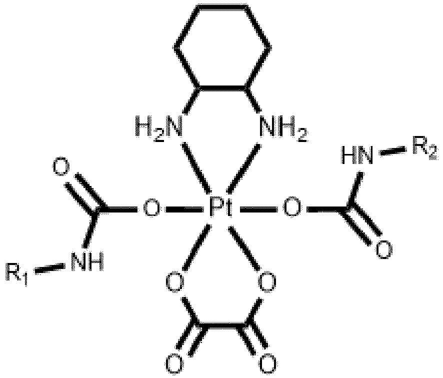 wherein R₁ und R₂ are the same or different branched or unbranched alkyls having at least 14 carbon atoms, a sonosensitizer and a biocompatible drug delivery system can be used to treat tumors and in particular metastases in a way that is well tolerated by the body. The prodrug nanoparticles according to the invention are particularly suitable for the treatment of mammary carcinomas, pancreatic carcinomas, prostate carcinomas, bronchial carcinomas, endometrial carcinomas, germ cell tumors, non-Hodgkin's lymphomas and malignant mesotheliomas and, in particular, colorectal carcinomas, and their respective metastases.

## Description

The present invention relates to a prodrug nanoparticle for tumor-targeted ultrasound-activated cancer therapy. The invention further relates to a formulation and a use of the prodrug nanoparticle.

Cancer is one of the most frequent causes of disease-related deaths worldwide and refers in particular to the development of a malignant neoplasm. Both the uncontrolled and increased proliferation of cells and the inhibition of naturally occurring apoptosis are due to various mutations or the deletion of genes. Malignant tumors can occur in various organs of the body and develop from different cell types. The tumor cells have invasive and migratory potential, allowing them to invade into healthy tissue and increasingly lead to its destruction. Single tumor cells or tumor cell clusters can also detach from the primary tumor and thus form local metastases, regional metastases or distant metastases via various metastasis pathways. When a cancer has formed metastases, its mortality is particularly high. The occurrence of the different types of cancer in the population depends, among other things, on the age, gender and territorial region of residence of the affected patients.

Colorectal carcinoma (cancer of the large intestine) is one of the most common types of cancer in both women and men. Colorectal carcinoma is a malignant neoplasm caused by the degeneration of epithelial cells. In 25 % of colorectal cancer patients, metastases have already manifested at the time of diagnosis. Metastases most frequently manifest in the liver (approx. 70 %), followed by the lungs, the non-regional lymph nodes or the peritoneum. In addition to resection of the primary tumor and subsequent radiotherapy treatment, chemotherapy is part of the standard treatment for metastatic colorectal cancer (mCRC).

During chemotherapy, cytostatic drugs are administered to the patient. Cytostatic drugs, however, are poorly tolerated and impose great strain on the body. Cytostatic drugs not only target tumor cells, but also healthy cells. Highly proliferating cells such as hair cells and mucosal cells are particularly affected. This can lead to frequent side effects such as hair loss, nausea, vomiting and serious side effects such as damage to peripheral sensory nerves or damage to blood formation with susceptibility to infections of all kinds, anaemia or coagulation disorders. The insufficient accumulation of cytostatic drugs in tumor tissue and emerging resistance mechanisms of tumor cells represent further limitations of antitumor chemotherapy.

Cytostatic drugs are cytotoxic chemical substances that interfere with the cell cycle and inhibit cell growth by restricting the cells' ability to divide. Platinum(II) complexes, in particular cisplatin, oxaliplatin and carboplatin, are among the most frequently used cytostatic drugs in cancer therapy. These compounds are administered in more than 50 % of all chemotherapeutic treatments worldwide. The cytostatic drugs must be administered in high doses in order to obtain a sufficient concentration in the tumor to be treated, resulting in the undesirable side effects described above.

It has been found that these disadvantages can be reduced if the platinum(ll) complexes are encapsulated in nanoparticles, resulting in a better selective accumulation of the platinum(II) complex in the tumor tissue. Due to the high proliferation rate of the tumor cells and the need for their own vascular system, intratumoral blood vessels are formed. Compared to normal tissue, intratumoral blood vessels often exhibit increased permeability of the capillary endothelium for larger particles. Due to the so-called *Enhanced Permeability and Retention* (EPR) effect, the platinum(II) complexes encapsulated in nanoparticles enter into the tumor tissue and selectively accumulate there, while the possibility for the nanoparticles to move into healthy tissue is greatly reduced. In addition, the platinum(II) complexes are protected from direct excretion via the kidneys by encapsulation in nanoparticles and are present in high concentrations until they reach the target site.

In order to reduce the side effects of cytostatic drugs, an alternative option is not to administer them to the body in their medically effective form, but to administer them as a precursor in the form of a prodrug. In the case of platinum, for example, it is not administered as platinum(II), but in the therapeutically inactive form of platinum(IV).

For example, "Liang G, Sadhukhan T, Banerjee S, Tang D, Zhang H, Cui M, Montesdeoca N, Karges J, Xiao H. Reduction of Platinum(IV) Prodrug Hemoglobin Nanoparticles with Deeply Penetrating Ultrasound Radiation for Tumor-Targeted Therapeutically Enhanced Anticancer Therapy. Angew Chem Int Ed Engl. 2023 May 22;62(22):e202301074. doi: 10.1002/anie.202301074. Epub 2023 Apr 19. PMID: 36961095." discloses a cisplatin(IV) prodrug that can be reduced to the therapeutically active cisplatin(II) form by ultrasound in the tumor microenvironment. Hemoglobin serves as a sonosensitizer and DSPE-PEG as a nanocarrier. This achieves a more targeted and reliable accumulation of the active drug at the target site. However, the stability of the nanoparticles still needs to be improved. Also, the selective accumulation of the cisplatin(IV) prodrug in the tumor tissue is based exclusively on the passive EPR effect, so that diffusion of the nanoparticles is not readily possible if the tumor's blood vessels are intact.

It is therefore an object of the present invention to create a stable prodrug nanoparticle that is well tolerated by the body, is inactive in healthy tissue and can be selectively activated in tumor tissue. Furthermore, it is an object of the present invention to provide a prodrug nanoparticle that can also be activated non-invasively in deep-seated tumors. In particular, it is also an object of the invention to create a chemical functionalization that ensures reliable and selective accumulation of a prodrug nanoparticle in tumor tissue. In addition, it is an object of the present invention to propose a targeted curative or palliative therapy for the treatment of a malignant tumor, in particular a colorectal carcinoma or its metastases. Furthermore, it is an object of the present invention to provide a formulation of such a prodrug nanoparticle as well as the use of said prodrug nanoparticle and of said formulation.

The object is achieved by a prodrug nanoparticle for targeted accumulation and activation (treatment) in tumor tissue, which consists of:
a) A Pt(IV) complex with the following formula: 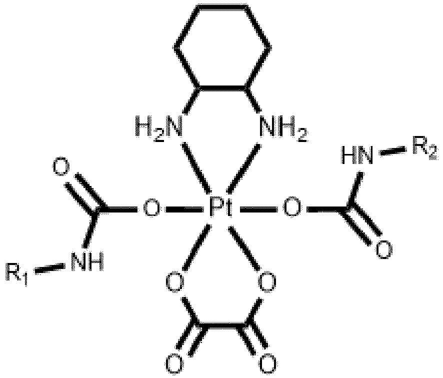 wherein R₁ und R₂ are the same or different branched or linear alkyls having at least 14 carbon atoms,
b) A sonosensitizer and
c) A biocompatible drug delivery system.

Surprisingly, it has been found that the prodrug nanoparticle according to the invention can be used to treat tumors and in particular metastases in a way that is well tolerated by the body. The prodrug nanoparticles according to the invention are particularly suitable for the treatment of mammary carcinomas, pancreatic carcinomas, prostate carcinomas, bronchial carcinomas, endometrial carcinomas, germ cell tumors, non-Hodgkin's lymphomas and malignant mesotheliomas and, in particular, colorectal carcinomas, and their respective metastases. The prodrug nanoparticles according to the invention accumulate selectively in a tumor such as a colorectal carcinoma and its metastases after intravenous injection. After irradiation with ultrasound, the platinum(IV) prodrug is activated in the tumor region, resulting in the release of cytotoxic oxaliplatin and almost complete eradication of the tumor. Colorectal carcinomas in particular form metastases in the liver, lungs, non-regional lymph nodes or peritoneum. Of these metastases, metastases in the lungs, liver and other regional metastases, even if they are localized deeper in the body, can be treated particularly well with the prodrug nanoparticles according to the invention. In other words, the present invention provides for a nanoformulated oxaliplatin-prodrug for spatially selective, ultrasound-activated anticancer therapy.

Compound a), the prodrug, is an oxaliplatin derivative in which platinum(IV) (Pt(IV)), in addition to 1,2-diaminocyclohexane and an oxalate ion, is complexed with two carbamate ligands, each of which has an aliphatic chain with at least 14 C atoms. Particularly preferably, the two carbamate ligands are the same carbamates; however, different carbamate ligands can also be used.

In an advantageous embodiment, the alkyls R₁ and/or R₂ each have from 14 to 24 carbon atoms, in particular from 14 to 20 carbon atoms. Linear alkyls are particularly preferred. Further preferred are branched alkyls with a main chain and one to four ethyl or, in particular, methyl groups.

Due to the encapsulation with the biocompatible drug delivery system c), in the prodrug nanoparticle, the inactive Pt(IV) complex a) (prodrug) and the sonosensitizer b) are present in close proximity to each other. This ensures spatial and temporal control over the activation to a therapeutic Pt(ll) complex form. The sonosensitizer enables activation by ultrasound and is therefore an "ultrasound antenna". The ultrasound radiation penetrates into deep tissue and also enables reliable reduction of the Pt(IV) complex there.

Due to the EPR effect, the prodrug nanoparticles selectively accumulate in the tumor microenvironment. In other words, the prodrug nanoparticles accumulate predominantly in the tumor tissue and only slightly in healthy tissue. Advantageously, the tumor region is selectively irradiated using ultrasound. In healthy tissue - even if the prodrug nanoparticles have accumulated there - the prodrug nanoparticles remain inactive. In tumor tissue, however, the Pt(IV) prodrug is activated by the ultrasound treatment and the Pt(IV) in the oxaliplatin derivative is reduced to Pt(II) by splitting off the two carbamate ligands; this process generates oxaliplatin, the pharmacologically active compound, which provides for the targeted treatment of the tumor and metastases. This applies in particular to colorectal carcinoma, mammary carcinoma, pancreatic carcinoma, prostate carcinoma, bronchial carcinoma, endometrial carcinoma, non-Hodgkin's lymphoma, malignant mesothelioma and germ cell tumors. Oxaliplatin is able to trigger immunogenic cell death. In addition to its direct effects on the tumor cells, oxaliplatin activates the immune system so that tumors and metastases are identified and eliminated by immune cells.

Encapsulation takes place by means of the biocompatible drug delivery system. Due to the long aliphatic hydrophobic chain, reliable physical encapsulation into nanoparticles is ensured. As a result, the Pt(IV) complex is stable and protected from direct excretion via the kidneys. Another advantage is that high concentrations of the drug can be used.

The prodrug nanoparticle of the present invention can be used to treat both primary tumors and, in particular, metastases. Metastasis refers to the detachment of tumor cells from the primary tumor and their invasion and migration into other tissues or organs. The resulting tumors nodules are called metastases. While the primary tumor can often be surgically removed, this is often not possible or only possible with difficulty in the case of metastases. The prodrug of the present invention is excellently suited for the treatment of metastases.

While the prodrug remains stable under physiological conditions, it is reduced in the presence of the sonosensitizer and under the influence of ultrasound radiation. Thus, the prodrug according to the invention remains stable and therefore inactive in healthy tissue but is rapidly reduced to the therapeutically active analog compounds in cancerous tissue upon ultrasound irradiation. In contrast to the previously performed chemical reduction of the prodrug by intracellular reducing agents (e.g. glutathione, ascorbic acid, metallothionein, thioredoxin), which are present in high concentrations in cancer cells, but also in healthy cells, the activation mechanism according to the invention is associated with good spatial and temporal control and a high reduction efficiency.

A particularly preferred embodiment is characterized in that the sonosensitizer is a tetrapyrrole, preferably a heme or a heme compound, in particular selected from the group consisting of hemoglobin, hemin b and hemin a. Other particularly preferred tetrapyrroles are in particular porphyrins, chlorins, bacteriochlorins and phthalocyanins. Cyanines are also particularly suitable as sonosensitizers.

The tetrapyrrole serves as a molecular ultrasound antenna. Advantageously, the immediate proximity of the tetrapyrrole to the Pt(IV) complex and the ultrasonic irradiation result in activation. This activation results in a reduction of the Pt(IV) complex to the medically active oxaliplatin with cleavage of the two carbamate ligands.

A preferred embodiment of the invention is also characterized in that the biocompatible drug delivery system is an amphiphilic drug delivery system, in particular poly(glycolic acid), poly(lactic acid), poly(lactic-co-glycolic acid), poly(ε-caprolactone), poloxamers, particularly preferably a DSPE-PEG polymer.

The drug delivery system has amphiphilic properties. The nanoparticle is lipophilic on the inside to ensure good interaction with the Pt complex and the sonosensitizer, while the outside is hydrophilic and thus well soluble in the blood plasma.

Examples of such amphiphilic drug delivery systems are polyethylene glycol (PEG), poly(glycolic acid), poly(lactic acid), poly(lactic-co-glycolic acid), poly(ε-caprolactone) and poloxamers.

DSPE-PEG ((1, 2-distearoyl-sn-glycero-3-phosphoethanolamine-poly(ethylene glycol)), including its ethoxylated and especially methoxylated variants, is particularly preferred as a biocompatible drug delivery system, as it is highly soluble in water. DSPE-PEG also reliably encapsulates all components of the prodrug nanoparticle. The DSPE-PEG particularly preferably has a molecular weight of 2,000 to 6,000 g/mol, especially preferably from 2,500 to 3,500 g/mol.

In a further advantageous embodiment, the DSPE-PEG is functionalized. Amines, carboxylic acids or maleimides are particularly suitable for functionalization.

A further preferred embodiment of the present invention is characterized in that the biocompatible drug delivery system, in particular DSPE-PEG, is functionalized with a tumor antigen-binding or tumor-associated antigen-binding domain, in particular with a peptide or an antibody.

This functionalization reliably ensures that the prodrug nanoparticle binds in the vicinity of the tumor, in particular in the tumor microenvironment. The tumor antigen, to which the tumor antigen-binding domain binds, is expressed on the tumor cells, and the tumor-associated antigen, to which the tumor-associated antigen-binding domain binds, is expressed on the cells of the tumor microenvironment. This has the advantage that the prodrug nanoparticle selectively accumulates in the tumor tissue and only to a small extent in healthy tissue. Particularly preferably, the tumor antigen-binding domain is a protein that is overexpressed on the cell surface of carcinoma cells, especially colorectal carcinoma cells, compared to normal tissue.

A further advantage is that this ensures active and selective accumulation in the tumor tissue. This additionally increases the selective accumulation in the tumor tissue due to the passive EPR effect.

In a further advantageous embodiment of the invention, the tumor-associated antigen-binding domain is directed against VEGFR2.

VEGFR2 is overexpressed on the endothelial cells of the intratumoral blood vessels compared to the endothelial cells outside the tumor microenvironment. The endothelial cells represent the first contact with the prodrug nanoparticle. By targeting the tumor-associated antigen-binding domain against VEGFR2, it is ensured that the prodrug nanoparticle binds predominantly in the direct tumor microenvironment. In addition to the passive EPR effect, this promotes accumulation within the tumor tissue. Another advantage is that VEGFR2 is temporarily blocked and thus the VEGF/VEGFR signal transduction pathway is inhibited, which reduces the formation of new blood vessels (neoangiogenesis) and thus tumor progression. This is particularly important in the progression of colorectal carcinoma as well as mammary carcinoma, pancreatic carcinoma, prostate carcinoma, bronchial carcinoma, endometrial carcinoma, non-Hodgkin's lymphoma, malignant mesothelioma and germ cell tumors.

According to a further preferred embodiment of the invention, the molar ratio of components a) to b) is from 1 : 4 to 20 : 1 , in particular from 1 : 2 to 10 : 1 and most preferably from 1 : 1 to 8 : 1 . The molar ratio of components a) to c) is preferably from 1 : 10 to 4 : 1 , in particular from 1 : 4 to 1 : 1 .

A preferred further embodiment of the invention is characterized by the fact that the prodrug can be activated to the active drug form by means of ultrasound. The targeted irradiation of the tumor region is an effective way of ensuring that the prodrug remains inactive in tumor-free tissue. In other words, the prodrug remains stable and therefore inactive in healthy tissue. Due to the use of ultrasound radiation, even deep-seated tumors can be reached. This ensures non-invasive treatment of tumors and metastases. Ultrasound irradiation rapidly reduces the prodrug in the tumor tissue by splitting off the two axial ligands into the therapeutically effective analog compound, oxaliplatin. This enables time and spatial control of the treatment. After reduction, the oxaliplatin is present in its therapeutically active form.

A further preferred embodiment of the invention is characterized in that, in addition to the oxaliplatin derivative, the nanoparticle is loaded with at least one further antitumoral substance, in particular a cytostatic agent or a combination of at least two cytostatic agents.

The additional loading of the nanoparticles with at least one antitumor substance thus extends the monotherapy to a combination therapy. The effect of the treatment is thus increased by synergistic effects of different antitumor substances. In particular, the nanoparticles are preferably loaded with antitumor substances that act through different mechanisms. Combination therapy reduces the likelihood of resistant tumor cells developing. Likewise, by combining the advantages of cytostatic combination therapy with the advantages of prodrug nanoparticles (selective accumulation and activation in the tumor), increased local anti-tumor activity is achieved with reduced systemic side effects. This makes a curative therapeutic approach possible in situations that were previously only accessible to palliative therapy (e.g. multiple or non-resectable liver metastases).

Still a further preferred embodiment of the invention is characterized in that the nanoparticle is additionally loaded with 5-fluorouracil and folinic acid, in particular analogous to the FOLFOX chemotherapy regimen.

FOLFOX refers to a chemotherapeutic treatment regimen for colorectal carcinoma, consisting of 5-fluorouracil, folinic acid and oxaliplatin. Treatment of metastatic colorectal carcinoma with FOLFOX is part of the therapeutic standard worldwide approved by relevant regulatory authorities and established in clinical practice. The additional use of 5-fluorouracil and folinic acid as part of the FOLFOX regimen improves the treatment results. In the proposed combination of the ultrasound-activated prodrug with other drugs according to the FOLFOX regimen, the possible side effects are limited by the selective accumulation in the tumor microenvironment and by the fact that the platinum(IV) complex is inactive in healthy tissue. Advantageously, FOLFOX serves as a curative and adjuvant therapeutic option. By combining the advantages of a cytostatic combination therapy (FOLFOX) with the advantages of prodrug nanoparticles (selective accumulation and activation in the tumor), an increased local anti-tumor activity with reduced systemic side effects is achieved. This makes a curative therapeutic approach possible in situations that were previously only accessible to palliative therapy (e.g. multiple or non-resectable liver metastases).

The object is also achieved by the above-mentioned formulation of the prodrug nanoparticle described above in the form of an infusion solution.

The formulation as an infusion solution offers the advantage that different application methods can be used. The formulation is particularly preferably injected intravenously or intraperitoneally. In the case of intravenous infusion, the formulation is highly bioavailable. The formulation reliably reaches the desired target site via the blood vessels. In the case of intraperitoneal infusion, the formulation acts locally with a high degree of effectiveness. This is particularly advantageous in the treatment of colorectal carcinoma and/or established intraperitoneal metastases.

The object of the present invention is also achieved by the aforementioned use of the prodrug nanoparticle described above or the formulation described above in cancer therapy, in particular for the treatment of tumor tissue, in particular colorectal carcinoma tissue and the metastases of the carcinoma. Colorectal carcinomas in particular form metastases in the liver, the lungs, the non-regional lymph nodes or the peritoneum. Of these metastases, metastases in the lungs, liver and other regionally limited metastases, even if they are localized deeper in the body, can be treated particularly well with the prodrug nanoparticles according to the invention.

It is particularly preferable to use the prodrug nanoparticle in conjunction with ultrasound irradiation of the tumor tissue to be treated. By irradiating, only the tumor tissue to be treated with ultrasound, the prodrug is only activated to the active substance there. In the non-irradiated areas, the prodrug remains in its inactive form, so that healthy tissue is not attacked, and undesirable side effects are reduced.

According to the present invention, treating colorectal carcinoma and its metastases, in particular in the liver, the lungs, the non-regional lymph nodes or the peritoneum, comprises a first step of administering to the patient the prodrug nanoparticle in a suitable form, in particular as an infusion. The tumor site is then selectively irradiated with ultrasound. The preferred frequency (or wavelength) of the ultrasound is from 0,1 MHz to 10 MHz, in particular from 1 MHz to 3 MHz.

The prodrug nanoparticle according to the present invention offers a reliable therapeutic approach in cancer therapy. When using the prodrug nanoparticle, already established therapeutic methods are applied, which lead to a considerably improved tolerability for the patient due to the activatable/reducible prodrug. This offers the advantage that, in contrast to conventional chemotherapies, its use has fewer effects on healthy tissue. The patient has significantly fewer side effects and therefore an improved quality of life during treatment.

The subject matter and the embodiments of the present invention are further illustrated in the drawings which serve to visualize aspects of the examples following hereinafter and in which
Figure 1 is a high-pressure liquid chromatography chromatogram (HPLC) of the individual components as well as the mixture of prodrugs with Hemin according to the examples inside of cell media upon exposure to ultrasound radiation (1.5 W/cm2) for various times;
Figure 2 depicts the size distribution of the prodrug nanoparticles according to the examples determined by dynamic light scattering; and
Figure 3 represents the drug concentration-response curves of the prodrug nanoparticles according to the examples, and oxaliplatin in the dark or upon exposure to ultrasound radiation in mouse colon carcinoma (CT-26) and human breast cancer (MCF-7) cells.

### Experimental

### Preparation of Pt(IV) Derivative of Oxaliplatin

### Example 1: [Pt(cyclohexane-1,2-diamine)(oxalic acid)(tetradecyl carbamate)₂] (Pt1)

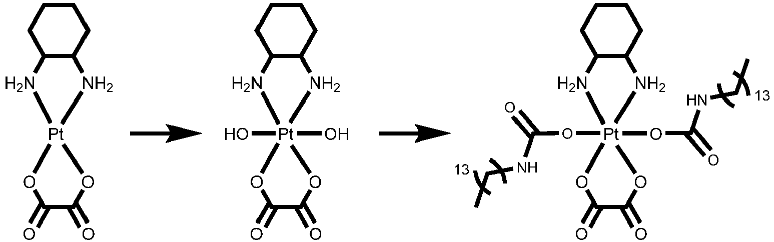

Oxaliplatin (1.65 mmol) was suspended in hydrogen peroxide (30%, 2.0 mL). The mixture was stirred at 40 °C overnight. After cooling down to room temperature, the product precipitated. The crude product was washed acetone and dried under reduced temperature.

Tetradecyl isocyanate (2.60 mmol) was added and the solution heated to 110 °C for 2 h. After this time, the reaction mixture was poured into ice water and the product precipitated. The solid was collected by filtration over filter paper and thoroughly washed with diethyl ether. The product was dried under vacuum. Yield: 68%.¹H-NMR (DMSO-d₆): δ = 6.71 (m, 4H), 6.25 (d, 2H), 2.93 (m, 4H), 2.74 (m, 2H), 2.21 (m, 4H), 2.17 (m, 4H), 1.23 (m, 48H), 0.85 (t, 6H) ppm; ESI-MS (positive mode) m/z 910.4 [M+H]⁺, calcd. C₃₈H₇₅N₄O₈Pt 910.5; Elemental analysis calcd. for C₄₂H₈₂N₄O₈Pt (%): C 50.15, H 8.20, N 6.16; found C 50.37, H 8.37, N 6.28; RP-HPLC: Rt = 13.7 min.

### Example 2: [Pt(cyclohexane-1,2-diamine)(oxalic acid)(hexadecyl carbamate)₂] (Pt2)

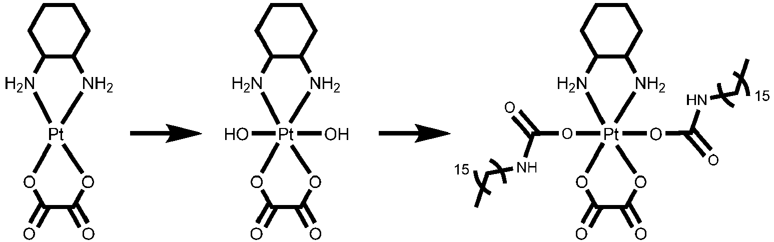

Oxaliplatin (1.65 mmol) was suspended in hydrogen peroxide (30%, 2.0 mL). The mixture was stirred at 40 °C overnight. After cooling down to room temperature, the product precipitated. The crude product was washed in acetone and dried under reduced temperature.

The oxidized oxaliplatin complex (1.30 mmol) was suspended in dimethylformamide (5 mL). Hexadecyl isocyanate (2.60 mmol) was added and the solution heated to 110 °C for 2 h. After this time, the reaction mixture was poured into ice water and the product precipitated. The solid was collected by filtration over filter paper and thoroughly washed with diethyl ether. The product was dried under vacuum. Yield: 53%. ¹H-NMR (DMSO-d₆): δ = 6.70 (m, 4H), 6.25 (d, 2H), 2.92 (m, 4H), 2.75 (m, 2H), 2.21 (m, 4H), 2.17 (m, 4H), 1.24 (m, 56H), 0.86 (t, 6H) ppm; ESI-MS (positive mode) m/z 966.5 [M+H]⁺, calcd. C₄₂H₈₃N₄O₈Pt 966.6; Elemental analysis calcd. for C₄₂H₈₂N₄O₈Pt (%): C 52.21, H 8.55, N 5.80; found C 52.07, H 8.31, N 5.73; RP-HPLC: Rt = 14.1 min.

### Reduction of the Pt(IV) Derivative of Oxaliplatin(IV) by means of Ultrasound Radiation

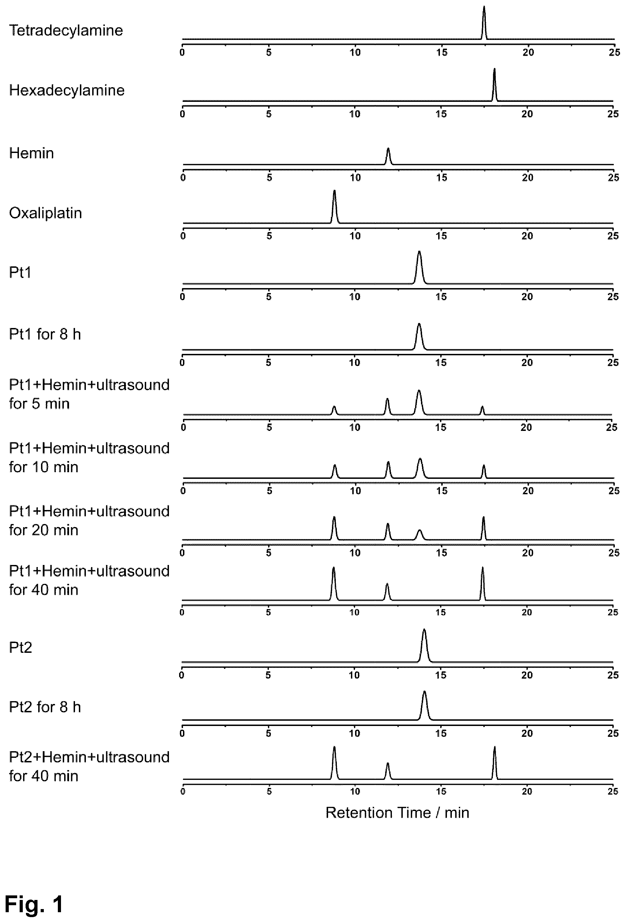

The ability to reduce the platinum(IV) prodrug was assessed upon incubation of the Oxaliplatin(IV) derivative with Hemin in cell media. The solution was then exposed to ultrasound radiation and afterwards analyzed by high-pressure liquid chromatography. The results show that upon incubation of the two components in water for up to 8 h, no decomposition or ligand release was observed. In contrast, upon exposure to ultrasound radiation the fast reduction of the Oxaliplatin(IV) prodrug into oxaliplatin as well as its axial ligand was observed (cf. Fig. 1).

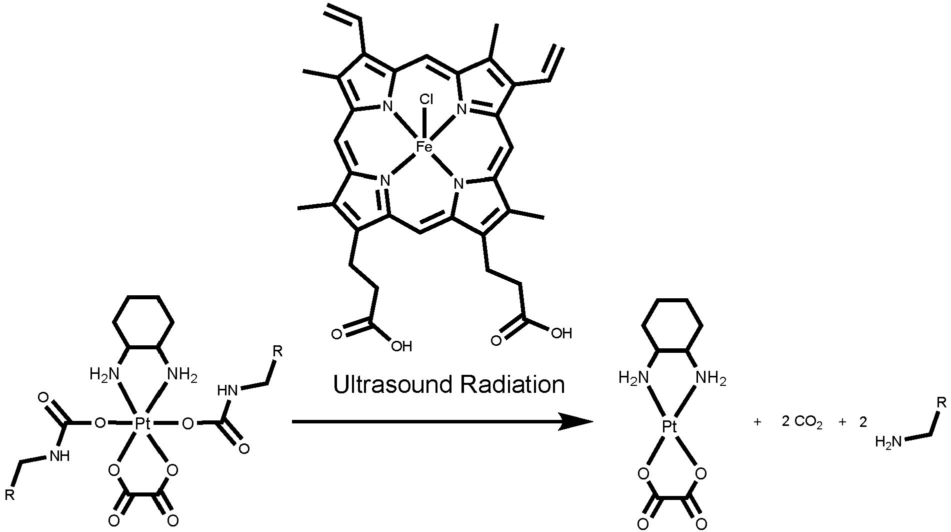

The high-pressure liquid chromatography chromatogram of the individual components as well as the mixture of Pt1 or Pt2 with Hemin inside of cell media upon exposure to ultrasound radiation (1.5 W/cm2) for various times, as depicted in Fig. 1 illustrates the occurrence of oxaliplatin and tetradecylamine and hexadecylamine, respectively, indicative of the release of the ligands and the reduction of Pt(IV) to Pt(II).

### Preparation of Nanoparticles from the Pt(IV) Derivative of Oxaliplatin (NP-Pt)

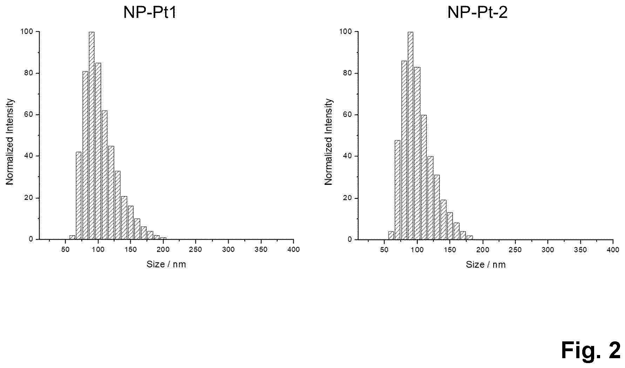

Hemoglobin (0.001 mmol) was dissolved in water (20 mL). A solution of Oxaliplatin(IV) (0.005 mmol) in dimethyl sulfoxide (0.5 mL) was added and the mixture vigorously stirred at room temperature. A solution of 1,2-distearoyl-sn-glycero-3-phospho-ethanolamine-n-[methoxy(polyethylene glycol)-2000 (DSPE-PEG₂₀₀₀-OMe, 0.01 mmol) in water (5 mL) was added to the mixture. The mixture was stirred at room temperature overnight. The solution was further purified by dialysis (MWCO: 3500 Da) for 24 h and the solution filtered through a 0.22 µm nylon membrane filter. The solvent was removed with a freeze-dryer and the product obtained as a powder. The size distribution of the nanoparticles was determined by dynamic light scattering and is shown in Fig. 2. The nanoparticle formulation generated from Pt1 is following referred to as NP-Pt1 and the formulation generated from Pt2 is following referred to as NP-Pt2. Fig. 2 demonstrates that the size distribution of the two nanoparticle formulations is very similar.

### Cytotoxicity of NP-Pt in the dark or upon exposure to ultrasound radiation

6 × 10³ cells were seeded onto 96-well plates and allowed to adhere overnight. The cells were treated with increasing concentrations of the compound which was diluted in cell media to a total volume of 200 µL. The cells were incubated with the compound for 4 h and after this time the medium refreshed. The cells for the dark group were grown for an additional 44 h at 37 °C. The cells for the ultrasound group were exposed to ultrasound radiation (1.5 W/cm², 2 min) with an Intelect Mobile 2 Ultrasound Emitter (DJO Global). The cells for the ultrasound group were grown for an additional 44 h at 37 °C. After this incubation time, the culture medium was substituted with PBS buffer containing 3-(4,5-dimethylthiazol-2-yl)-2,5-diphenyltetrazolium bromide (MTT) with a final concentration of 12 µM. The cells were incubated for 2 h at 37 °C and 10 % CO₂ After this time, the DPBS-MTT mixture was removed and the wells filled with 200 µL DMSO. Using a 620 nm reference wavelength, the absorption of the formazan was measured at 550 nm with an Infinite M Nano plus Microplate reader (Tecan).

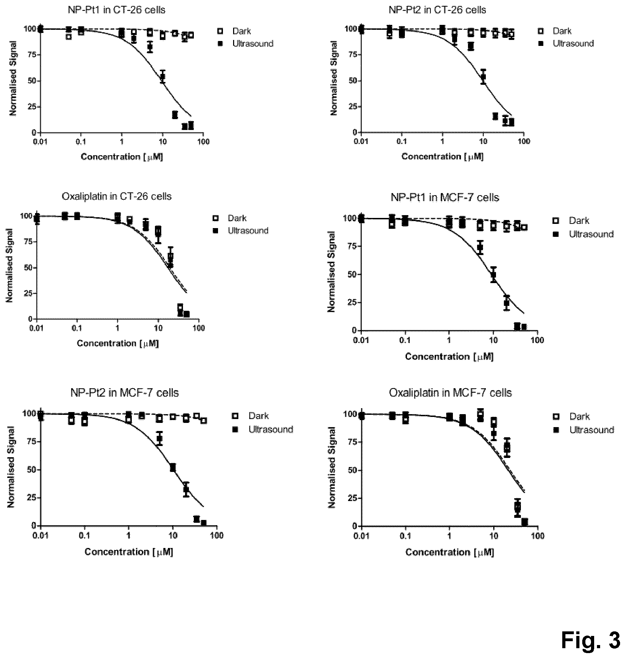

The nanoparticles were found to be non-toxic in the dark (IC50 > 50 µM), i. e. without irradiation. In contrast, the nanoparticles induced a cytotoxic effect upon exposure to ultrasound radiation. Table 1 reflects the respective data, which are also visualized in Fig. 3.

**Table 1: Cytotoxicity (IC50 in µM) of oxaliplatin, NP-Pt-1 and NP-Pt-2 in the dark or upon exposure to ultrasound radiation in mouse colon carcinoma (CT-26) and human breast cancer (MCF-7) cells. The mentioned concentrations correspond to the platinum complex.**

| | CT-26 | | MCF-7 | |
|---|---|---|---|---|
| | dark | ultrasound | dark | ultrasound |
| NP-Pt-1 (tetradecyl carbamate) | >50 | 9.6 ± 0.8 | >50 | 9.2 ± 0.7 |
| NP-Pt-2 (hexadecyl carbamate) | >50 | 9.8 ± 0.7 | >50 | 10.1 ± 0.9 |
| Oxaliplatin | 20.4 ± 3.1 | 19.3 ± 3.4 | 24.5 ± 4.6 | 22.4 ± 4.8 |

In addition, Fig. 3 demonstrates the increasing toxicity of oxaliplatin for increasing concentrations. Unlike oxaliplatin, the prodrug nanoparticles of the present invention exhibit a very low toxicity in the dark, i. e. in the non-activated state, even for high concentration. As activation is only effected in the tumor, while the prodrug remains inactive in other, i. e. healthy, tissue, no toxic effect will be provoked in said healthy tissue.

## Claims

1. Prodrug nanoparticle for the targeted accumulation and activation in tumor tissue, that consists of:
a) a Pt(IV) complex having the following formula: 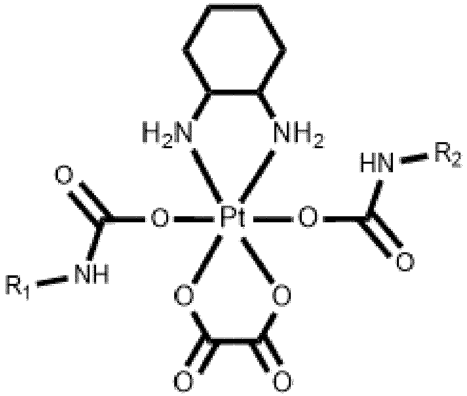 wherein R₁ und R₂ are the same or different branched or linear alkyls having at least 14 carbon atoms,
b) a sonosensitizer and
c) a biocompatible drug delivery system.

2. Prodrug nanoparticle according to claim 1, **characterized in that** the sonosensitizer is a cyanine or a tetrapyrrole, preferably a heme or a heme compound, in particular selected from the group consisting of hemoglobin, hemin b and hemin *a*.

3. Prodrug nanoparticle according to claim 1 or 2, **characterized in that** the biocompatible drug delivery system is an amphiphilic drug delivery system, in particular poly(glycolic acid), poly(lactic acid), poly(lactic-co-glycolic acid), poly(ε-caprolactone), poloxamers, particularly preferably a DSPE-PEG polymer.

4. Prodrug nanoparticle according to claim 3, **characterized in that** the DSPE-PEG is functionalized with a tumor antigen-binding or tumor-associated antigen-binding domain, in particular with a peptide or an antibody.

5. Prodrug nanoparticle according to claim 4, **characterized in that** the tumor-associated antigen-binding domain is directed against VEGFR2.

6. Prodrug nanoparticle according to one of claims 1 to 5, **characterized in that** the ratio of components a) to b) is from 1 : 4 to 20 : 1 , in particular from 1 : 2 to 10 : 1 and most preferably from 1 : 1 to 8 : 1.

7. Prodrug nanoparticle according to one of claims 1 to 6, **characterized in that** the prodrug can be activated to the active drug by means of ultrasound.

8. Prodrug nanoparticle according to one of claims 1 to 7, **characterized in that** the platinum (IV) of the prodrug is converted intratumorally into platinum (II) of the therapeutically active drug.

9. Prodrug nanoparticle according to one of claims 1 to 8, **characterized in that** the nanoparticle is additionally loaded with at least one further antitumoral substance, in particular a cytostatic agent or a combination of at least two cytostatic agents.

10. Prodrug nanoparticle according to claim 9, **characterized in that** the nanoparticle is additionally loaded with 5-fluorouracil and folinic acid, in particular analogous to the FOLFOX chemotherapy regimen.

11. Prodrug nanoparticle according to one of claims 1 to 10, **characterized in that** it is suitable for the treatment of mammary carcinomas, pancreatic carcinomas, prostate carcinomas, bronchial carcinomas, endometrial carcinomas, germ cell tumors, non-Hodgkin's lymphomas and malignant mesotheliomas and, in particular, colorectal carcinomas and their respective metastases.

12. Formulation of the prodrug nanoparticle according to one of claims 1 to 11 in the form of an infusion solution.

13. Use of the prodrug nanoparticle according to one of claims 1 to 11 or of the formulation according to claim 12 in cancer therapy, in particular for the treatment of tumor tissue, particularly for the treatment of mammary carcinomas, pancreatic carcinomas, prostate carcinomas, bronchial carcinomas, endometrial carcinomas, germ cell tumors, non-Hodgkin's lymphomas and malignant mesotheliomas and, in particular, colorectal carcinomas and their respective metastases.

14. Use according to claim 13, **characterized in that** the use includes ultrasound irradiation of the tumor tissue to be treated subsequent to administration of the prodrug nanoparticle.
